(19) Europäisches Patentamt
European Patent Office
Office européen des brevets

(11) EP 4 549 619 A1

(12) EUROPEAN PATENT APPLICATION

(43) Date of publication:
**07.05.2025 Bulletin 2025/19**

(21) Application number: **24207987.9**

(22) Date of filing: **22.10.2024**

(51) International Patent Classification (IPC):
***C25B 1/04*** *(2021.01)* ***C25B 15/08*** *(2006.01)*
***C01B 4/00*** *(2006.01)* ***C07B 59/00*** *(2006.01)*
***C07C 209/34*** *(2006.01)* ***C07C 209/36*** *(2006.01)*

(52) Cooperative Patent Classification (CPC):
**C25B 1/04; C01B 4/00; C07B 59/001; C07C 209/34; C07C 209/36; C25B 15/081;**
C07B 2200/05

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 EP 23206872**

(71) Applicant: **BASF SE**
**67056 Ludwigshafen am Rhein (DE)**

(72) Inventors:
- **Vossen, Marcus**
  **67117 Limburgerhof (DE)**
- **Stock, Christoph**
  **67056 Ludwigshafen am Rhein (DE)**
- **Ernst, Martin**
  **67056 Ludwigshafen am Rhein (DE)**
- **Ulrich, Viktor**
  **67056 Ludwigshafen am Rhein (DE)**
- **Harhausen, Sina**
  **67056 Ludwigshafen am Rhein (DE)**
- **Xiong, Jiawen**
  **67056 Ludwigshafen am Rhein (DE)**
- **Hueffer, Stephan**
  **67063 Ludwigshafen (DE)**
- **Krueger, Marco**
  **67056 Ludwigshafen am Rhein (DE)**
- **Weiss, Thomas**
  **67056 Ludwigshafen am Rhein (DE)**

(74) Representative: **BASF IP Association**
**BASF SE**
**GBI - Z078**
**67056 Ludwigshafen (DE)**

# (54) PREPARATION OF AMINES BY HYDROGENATION OF NITRO COMPOUNDS USING HYDROGEN WITH LOW DEUTERIUM CONTENT

(57) A process for the preparation of an amine compound comprising the following steps:
(a) providing hydrogen with a molar share of deuterium ≤ 100 ppm, based on the total hydrogen content, by electrolysis of water using electrical power generated at least in part from non-fossil energy,
(b) at least partially hydrogenating a nitro compound to form the corresponding amine compound.

EP 4 549 619 A1

## Description

The present invention relates to a hydrogenation process for the preparation of amines by hydrogenation of nitro compounds using hydrogen. The obtained amines have a lower deuterium content, based on the hydrogen content in the amino group, compared to corresponding amines generated by reaction with hydrogen that is produced from petrochemical processes using natural gas, condensate or petroleum oil. The invention further relates to the amine compounds obtained thereby as well as to their use.

In the preparation of amines by hydrogenation of nitro compounds via hydrogenation, hydrogen and in most cases a catalyst is used.

In modern industrial processes, a significant amount of the hydrogen is provided by steam reforming, thus, from natural gas. However, the petrochemical steam reforming process has its negative impacts regarding its carbon footprint including the consumption of a lot of fossil-based natural resources and energy.

It is therefore an objective of the present invention to provide environmentally friendly amines in an environmentally friendly process for making the same, that process using as little fossil-based energy as possible.

The object is achieved by a process for the preparation of amines comprising the following steps:

(a) providing hydrogen with a molar share of deuterium $\leq$ 100 ppm, based on the total hydrogen content, by electrolysis of water using electrical power generated at least in part from non-fossil energy,
(b) at least partially hydrogenating a nitro compound to form the corresponding amine.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1$H and deuterium $^2$H). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest since the effect is related to vibrational frequencies of the affected bonds. For instance, changing a hydrogen atom (H) to its isotope deuterium (D) represents a 100 % increase in mass, whereas in replacing $^{12}$C with $^{13}$C, the mass increases by only 8 percent. The rate of a reaction involving a C-H bond is typically 6-10 times faster than the corresponding C-D bond, whereas a $^{12}$C reaction is only 4 percent faster than the corresponding $^{13}$C reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general $\leq$ 100 ppm, preferably in general $\leq$ 90 ppm, for example from 30 to 75 ppm. The deuterium atom content in electrolytically produced hydrogen may be as low as 10 ppm. The deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline

electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 $mA/cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nafion®, fumapem®) are used as an electrolyte (proton conductor). These proton exchange membranes have many advantages such as low gas permeability, high proton conductivity ($0.1 \pm 0.02$ S $cm^{-1}$), low thickness (20-300 μm), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2/RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 μm) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nafion®, a DuPont product. While Nafion® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor β

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A $cm^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), ρ is the density of water (g $mL^{-1}$) and $M_{H2O}$ (g $mol^{-1}$) is the molar weight of water. A stoichiometric number A of 10 means that 10 times the amount of fresh water than can be theoretically consumed by electrolysis at the given electrolysis current is

supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%.

The electrical power from non-fossil resources used in water electrolysis according to the invention can be generated by nuclear energy. Nuclear energy is considered renewable by the European Commission, as long as certain preconditions (inter alia safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary. Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare

with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

In step (b), a nitro compound is hydrogenated using the hydrogen provided in step (a) to form the corresponding amine compound.

Preferred nitro compounds to be hydrogenated in step (b) are aliphatic, heteroaliphatic (i. e. containing skeleton hetero atoms, preferably nitrogen atoms in the aliphatic chain), heterocyclic (preferably containing oxygen or nitrogen hetero atoms) or aromatic or heteroaromatic nitro compounds containing a total of from 2 to 20 carbon atoms and from 1 to 3 nitro groups.

The nitro compounds to be hydrogenated in step (b) can contain from 1 to 5, preferably from 1 to 3 further functional groups selected from hydroxyl groups, carbonyl groups, nitrile groups, ether groups and amino groups. Carbonyl groups can be keto groups or can be part of aldehyde groups, carboxylic acid groups, carboxylic acid ester groups and dicarboxylic acid anhydride groups. Carbonyl groups, in particular aldehyde groups, may also be hydrogenated in the process of the invention.

The nitro compounds to be hydrogenated in step (b) can contain from 1 to 5, preferably from 1 to 3 halogen atoms, preferably chlorine or fluorine atoms.

In certain preferred embodiments, the nitro compounds to be hydrogenated are aliphatic or heteroaliphatic or heterocyclic nitro compounds containing a total of from 3 to 16 carbon atoms, 1 or 2 nitro groups, and optionally 1 to 3 further functional groups selected from hydroxyl groups and amino groups. A particular subgroup of these compounds are aliphatic mononitro compounds containing a total of 3 to 6, preferably 4 or 5 carbon atoms, and 1 to 3 further functional groups selected from hydroxyl groups and amino groups. A further particular subgroup of these nitro compounds are heteroaliphatic dinitro compounds containing 12 to 18, preferably 14 to 16 carbon atoms, 1 or 2 skeleton nitrogen atoms (bound in the form of secondary or tertiary amino groups) in the aliphatic chain, and 1 to 3 hydroxyl groups.

In certain further preferred embodiments, the nitro compounds to be hydrogenated are aromatic or heteroaromatic nitro compounds containing a total of from 6 to 14 carbon atoms, 1 or 2 nitro groups and optionally 1 or 2 halogen atoms selected from chlorine and fluorine.

The aromatic or heteroaromatic core will not be hydrogenated in the process of the invention.

The process of hydrogenating nitro compounds into amine compound can be carried out in various ways, which can be differentiated according to the type of catalyst, the feedstock and the physical state of the reactants. See: C.Stock (2023) Hydrogenation and Dehydrogenation; Chapter 1.9.8 Hydrogenation of Nitro Compounds; in Ullmann's Encyclopedia of Industrial Chemistry (https://doi.org/10.1002/14356007.a13_487.pub3).

Hydrogenation of aromatic nitro compounds

Historically, aniline was produced by reduction of nitrobenzene with iron under acidic conditions "B6champ Reduction" [Natte, K., Jagadeesh, R., (2021) Kirk-Othmer Encyclopedia of Chemical Technology, Chapter Amines by Reduction, John Wiley and Sons, New York]. This process was replaced by the heterogeneously catalyzed hydrogenation of nitrobenzene using copper on silica as catalyst at 270-300 °C and 1-10 bar in a gas phase hydrogenation reaction. In the industrial process, a fluidized catalyst bed is used. [Wittcoff H. A., Reuben, B. G., Plotkin, J. S. (2004) Industrial Organic Chemicals, Wiley, 2nd Edition, Chapter 7, 294 or EP3953322 A1 BASF SE].

Alternatively, the hydrogenation of gaseous nitrobenzene is catalyzed over supported noble metal catalysts such as Pd [EP0011090 B1 BAYER AG]. Also fixed bed catalysts based on supported base or noble metals are known for the hydrogenation of nitrobenzene at 200-410 °C, 4-18 bar and adiabatic conditions [EP4021885A1 Covestro and EP0696573 B1 BAYER AG]. Aniline can be also produced in liquid-phase nitrobenzene hydrogenation with a slurry Pt containing catalyst [CN112108139]. Typical operation conditions for the liquid-phase hydrogenation conditions are high-pressure hydrogenation of 16-18 bar and temperatures between 50 and 100 °C [CN113019270].

Toluidine and toluyldiamine are produced via hydrogenation of the corresponding nitro- or dinitro-compound using supported nickel catalysts in a slurry hydrogenation [Ullmann's Enzyklopädie der technischen Chemie, 4. edition, volume 7, pp 393 ff, 1973, Verlag Chemie, Weinheim/New York]. Typical reaction conditions are 80-140 °C and 10-30 bar. Syntheses of these aromatic amines are also known to be catalyzed by noble metal catalysts at temperatures above 185 °C and 10-30 bars [EP2382181 BASF SE]. Alternatively, the reduction is performed in a vapor phase process using nickel, Raney Ni, Pd/C, Pt/C catalysts and temperatures of 170-350 °C and pressures of 20-100 bar [EP3055284 Covestro].

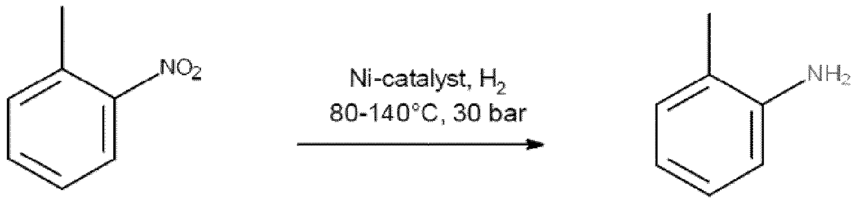


Diaminonaphthalene is formed via hydrogenation of dinitronaphthalene over supported Pd or Pt catalysts at 30-150°C and 4-40 bar [CN101575295 and DE2438542 BAYER AG].

Halogenated nitroaromatics such as chloroaniline or fluoroaniline can be prepared over supported Pd and/or skeletal Ni catalyst at 50-150 °C and 5-12 bar or over supported Pt at 120-280°C, respectively [CN103664642 Sinopec and CN104130129]. Furthermore, synthesis of dichloroaniline by dichloronitrobenzene hydrogenation is carried out over either base or noble metal catalyst at temperatures between 80-200°C and 3-30 bar [EP0398542 DuPont and CN103694124 Sinopec].

Hydrogenation of aliphatic nitro compounds

Nitroalkanes are commercially available or can be readily prepared by those skilled in the art using well-known literature techniques. These are hydrogenated to the corresponding alkylamines.

Examples reactions are:

Hydrogenation of 2-Nitro-2-methyl-1-propylamine to 2-Methyl-1,2-diaminopropane (as described in US2012/038336, Angus Chemical Co.):

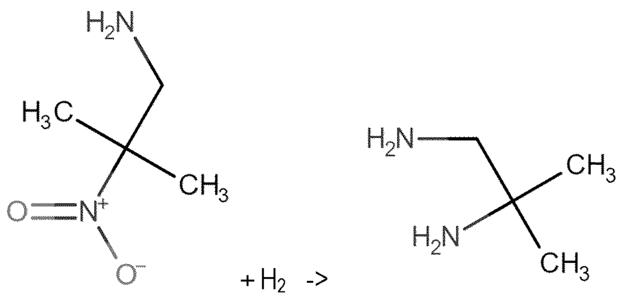


Likewise, nitroalkanols are hydrogenated to yield the corresponding aminoalcohols.

Aminoalcohols play an important role in a variety of commercial and consumer products. For instance, they are used as neutralizers in paints and coatings or in personal care products.

The hydrogenation reaction is carried out in the presence of hydrogen gas in combination with a hydrogenation catalyst, for example Raney Nickel or a platinum or palladium-based catalyst (Pt or PD in elemental form or as oxides, with or without supports e.g. carbon) (WO 2011/115803 A1, Angus Chemical Co.).

Examples reactions are:

Hydrogenation of 2-nitro-butanol to 2-amino-butanol (as described in WO 2011/115803 A1, Angus Chemical Co.):

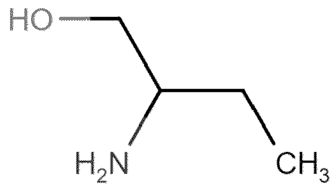

Hydrogenation of 2-nitro-2-methylpropanol to 2-amino-2-methylpropanol (as described in WO 2011/115803 A1, Angus Chemical Co.):

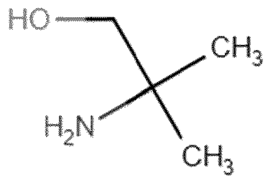

Hydrogenation of 2-nitro-2-ethyl-1,3-propandiol to 2-amino-2-ethyl-1,3-propandiol (as described in WO 2011/115803 A1, Angus Chemical Co.):

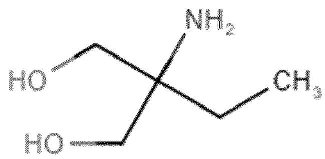

Hydrogenation of 2-nitro-2-hydroxymethyl-1,3-propandiol to 2-amino-2-hydroxymethyl-1,3-propandiol (as described in WO 2011/115803 A1, Angus Chemical Co.):

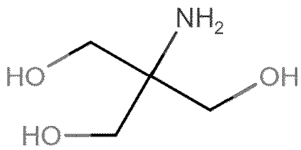

Hydrogenation of 2-nitro-2-methyl-1,3-propandiol to 2-amino-2-methyl-1,3-propandiol (as described in WO 2011/115803 A1, Angus Chemical Co.):

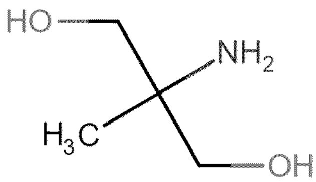

Hydrogenation of 2-nitro-1,3-propandiol to 2-amino-1,3-propandiol (as described in EP 0 348 223 A2, W.R Grace & Co.):

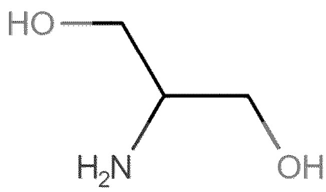

Hydrogenation of 1,1'-(ethane-1,2-diylbis (methylazanediyl))bis(4-nitro-4-methylpentan-2-ol) to form 1,1'-(ethane-1,2-diylbis(methylazanediyl))bis(4-amino-4-methylpentan-2-ol) (as described in US 8,480,800 B2, Angus / DOW):

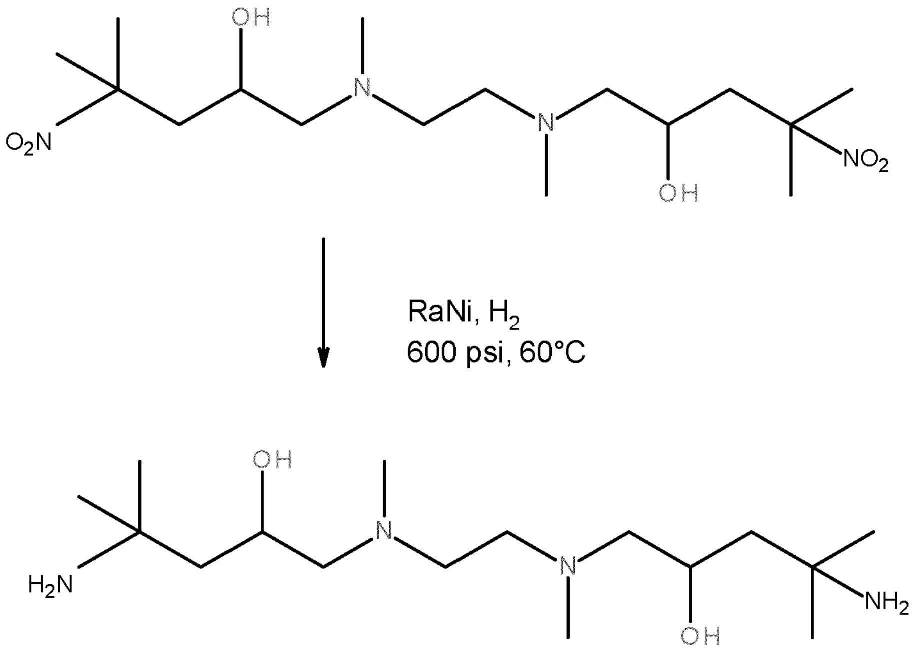


Hydrogenation of 1,1'-(2-hydroxyethylazanediyl)bis(4-nitro-4-methylpentan-2-ol) to form 1,1'-(2-hydroxyethylazanediyl)bis(4-amino-4-methylpentan-2-ol) (as described in US 8,480,800 B2, Angus / DOW):

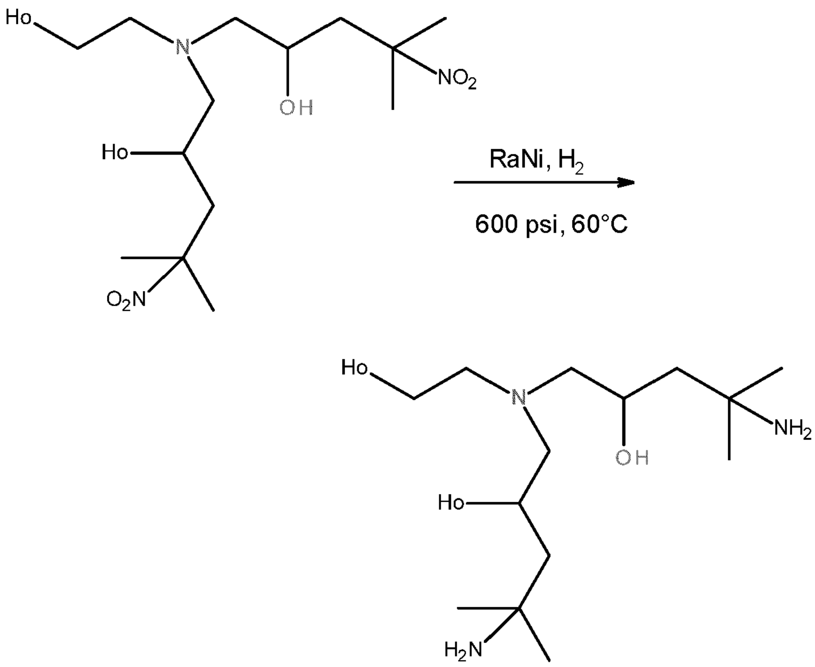


The 5-nitro-1,3-dioxane derivative (III) is hydrogenated to form the corresponding 5-amino-1 ,3-dioxane derivative (IV) (as described in EP 0 348 223 A2):

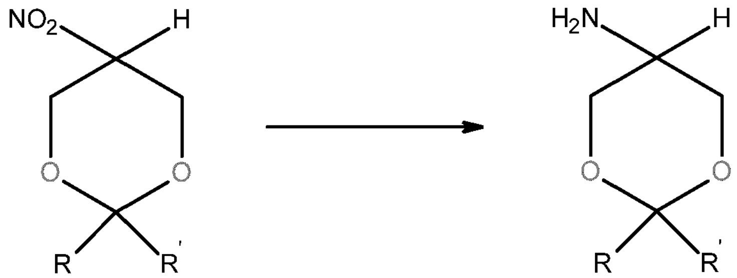

Most preferred are the following hydrogenations (1) to (17).

(1) nitrobenzene to aniline;
(2) nitrotoluene to toluidine;
(3) dinitrotoluene to toluyldiamine;
(4) dinitronaphthalene to diaminonaphthalene;
(5) chloronitrobenzene to chloroaniline;
(6) fluoronitrobenzene to fluoroaniline;
(7) dichloronitrobenzene to dichloroaniline;
(8) 2-nitro-2-methyl-1-propylamine to 2-methyl-1,2-diaminopropane;
(9) 2-nitro-butanol to 2-amino-butanol;
(10) 2-nitro-2-methylpropanol to 2-amino-2-methylpropanol;
(11) 2-nitro-2-methyl-1,3-propandiol to 2-amino-2-methyl-1,3-propandiol;
(12) 2-nitro-2-ethyl-1,3-propandiol to 2-amino-2-ethyl-1,3-propandiol;
(13) 2-nitro-2-hydroxymethyl-1,3-propandiol to 2-amino-2-hydroxymethyl-1,3-propandiol;
(14) 1,1'-(ethane-1,2-diylbis(methylazanediyl))bis(4-nitro-4-methylpentan-2-ol) to 1,1'-(ethane-1,2-diylbis(methylazanediyl))bis(4-amino-4-methylpentan-2-ol);
(15) 1,1 '-(2-hydroxyethylazanediyl)bis(4-nitro-4-methylpentan-2-ol) to form 1,1'-(2-hydroxyethylazanediyl)bis(4-amino-4-methylpentan-2-ol);
(16) 5-nitro-1,3-dioxane derivatives to the corresponding 5-Amino-1,3-dioxane derivatives.
(17) p-nitro-benzoic-acid-2-ethylhexyl-ester to p-amino-benzoic-acid-2-ethylhexyl-ester

Regarding the reaction mentioned under (17) above, the reaction is conducted in the presence of a catalyst, wherein the catalyst is preferably a precious metal catalyst, preferably selected from palladium, Raney Nickel, rhodium, platinum, or mixtures thereof. The hydrogenation reaction can be performed in different solvents, for example in ethanol, methanol, water, mixtures thereof or in a mixture of water and 2-ethylhexanol. The temperature for the hydrogenation reaction is preferably selected to be in the range of from 50°C to 120°C. The aromatic amine compound obtained in this manner, p-amino benzoic acid 2-ethylhexyl ester, may be further reacted with cyanuric chloride in a ratio of 3:1 to furnish ethylhexyl triazone, a UV absorber. The hydrogenation of p-nitro benzoic acid 2-ethylhexyl ester and the subsequent use of the amine compound to provide ethylhexyl triazone is also described in EP 3 674 293 A1.

The invention also relates to the amine compounds that can be obtained by the process of the invention. Included are amine compounds that result from partial or full hydrogenation of the respective nitro compounds. Partially hydrogenated nitro compounds can still contain (remaining) nitro groups.

Beside the hydrogenation of nitro compounds, also nitrogen monoxide as such can be hydrogenated to hydroxyl amine with hydrogen having a molar share of deuterium ≤ 100 ppm, based on the total hydrogen content, and being obtained by electrolysis of water using electrical power generated at least in part from non-fossil energy. The reaction scheme is as follows:

$$2\ NO + 3H_2 \rightarrow 2\ NH_2OH$$

Preferably, the amine compounds obtainable according to the invention are aliphatic, heteroaliphatic, heterocyclic or aromatic or heteroaromatic amine compounds containing a total of from 2 to 20 carbon atoms and 1 to 3 primary amino groups. The amine compounds can contain 1 to 5, preferably 1 to 3 further functional groups selected from hydroxyl groups, carbonyl groups, nitrile groups and ether groups. The amine compounds can contain 1 to 5, preferably 1 to 3 halogen atoms, preferably selected from chlorine and fluorine atoms.

The inventive amine compounds are characterized by the molar share of deuterium in the hydrogen bound in the primary amino group as a result of step (b) of the process according to the invention of ≤ 100 ppm, preferably in the range of from 10 to 95 ppm, more preferably in the range of from 15 to 90 ppm, most preferably in the range of from 20 to 80 ppm,

especially in the range of from 30 to 75 ppm, based on the total content of said hydrogen.

Said low deuterium molar share is introduced by step (a) of the process according to the present invention. With the present invention environmentally friendly amine compounds and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The downstream products based on hydrogen obtained by electrolysis can be distinguished by their deuterium molar share from amine compounds based on hydrogen prepared by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are amine compounds.

The present invention further relates to a process for tracing the origin, especially the energetic origin, of hydrogen bound in downstream compounds based on hydrogen by determining the molar share of deuterium in said downstream compounds based on hydrogen, wherein the downstream compounds are amine compounds.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Furthermore, the products according to the process described herein can be converted to a product as described on page 18, line 29 to page 26, line 29 of European patent application No. EP24164893.0.

## Examples

### I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell: Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nafion® 117 standard membrane (supplier DuPont, dry thickness 180 microns) was assembled by HIAT GmbH with catalytic active coating materials iridium (19 $g/m^2$) and platinum (8 $g/m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell: Alkaline water electrolysis was conducted with a circular AEC electrolysis cell (model Electro MP Cell, supplier ElectroCell Europe A/S, 0.01 $m^2$ electrode area). As electrodes Nickel 2.4068 material was used and

separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell: The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station: As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.

The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

The results of the hydrogen production are shown in Table 1.

II Further examples

Synthesis of benzotriazoles such as for example 2-(2H-benzotriazol-2-yl)-4,6-bis(1-methyl-1-phenylethyl)phenol (brand name Tinuvin 234) according to the following Scheme.

The process for preparing benzotriazoles from o-nitroaniline (or p-Chloro-o-Nitroaniline) is carried out in 3 reaction steps, which are shortly outlined below:

a) Diazotization of o-nitroaniline or p-choro-o-nitroaniline

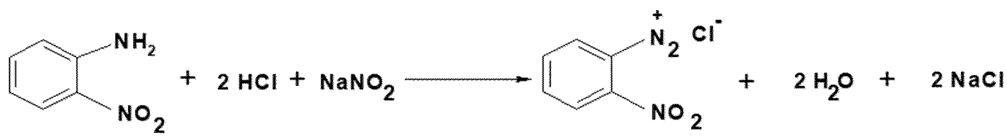


b) Coupling of diazo-compound a) on a substituted phenol to obtain an azo-compound

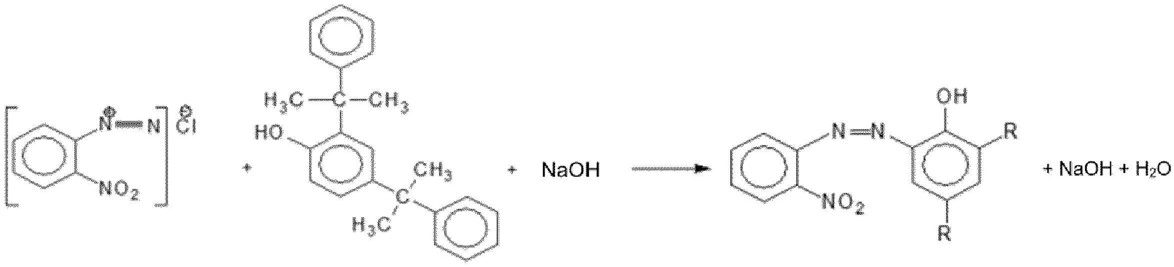


whereas the substituted phenol can generally be a compound as shown below

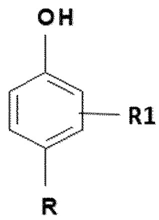

R = Methyl, tert-Butyl, tert-Octyl, Dimethylbenzyl, tert-Amyl, Propanoic acid ester (isooctyl, polyethyleneglycol, n-Octyl, 2-Ethylhexyl)
R1 = sec.Butyl, tert-Butyl, Dodecyl, Dimethylbenzyl, tert-Amyl,

and with

R = 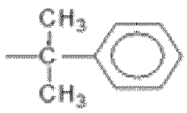

in case of the production of Tinuvin 234.

c) Reduction of the azo-compound b) with hydrogen to benzotriazole in presence of a catalyst at a temperature between 20°C and 100°C and at a pressure of 1 to 100 bar followed by work-up

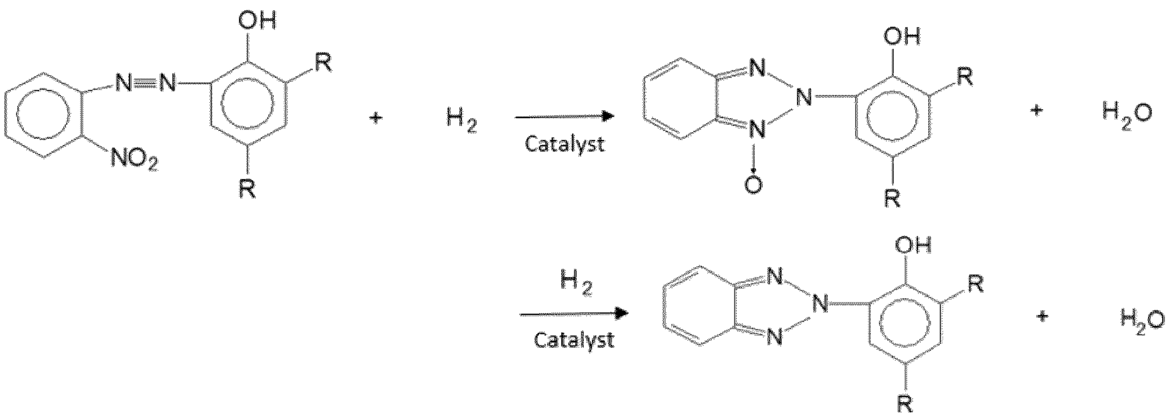


whereas in case of Tinuvin 234 with the chemical name 2-(2H-benzotriazol-2-yl)-4,6-bis(1-methyl-1-phenylethyl)phenol, the substituent R is

R = 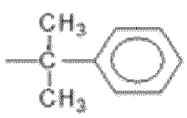

Table 1

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |

* Isar river surface water (Munich) collected in winter season January 2021.

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples (amine compounds) was executed via isotope ratio monitoring gas chromatography/mass spectrometry (IRMS). Therefore a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^2$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Production of amine compounds

Amine compounds are prepared in industrial scale synthesis processes as described above.

## Claims

**1.** A process for the preparation of an amine compound comprising the following steps:

(a) providing hydrogen with a molar share of deuterium ≤ 100 ppm, based on the total hydrogen content, by electrolysis of water using electrical power generated at least in part from non-fossil energy,

(b) at least partially hydrogenating a nitro compound to form the corresponding amine compound.

2. The process of claim 1, wherein the electrical power is generated from wind power, solar energy, biomass, hydropower and geothermal energy.

3. The process according to claim 1 or 2, wherein hydrogen is provided by polymer electrolyte membrane water electrolysis.

4. The process according to claim 3, wherein hydrogen is provided by proton exchange membrane water electrolysis (PEMWE) or anion exchange membrane water electrolysis (AEMWE).

5. The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

6. The process according to any one of claims 1 to 5, wherein step (c) is carried out as heterogeneously or homogeneously catalyzed reaction.

7. The process according to any one of claims 1 to 6, wherein the nitro compounds to be hydrogenated in step (b) are aliphatic, heteroaliphatic, heterocyclic or aromatic or heteroaromatic nitro compounds containing a total of from 2 to 20 carbon atoms and 1 to 3 nitro groups.

8. The process according to claim 7, wherein the nitro compounds to be hydrogenated in step (b) contain 1 to 5, preferably 1 to 3 further functional groups selected from hydroxyl groups, carbonyl groups, nitrile groups, ether groups and primary amino groups.

9. The process according to claim 7 or 8, wherein the nitro compounds to be hydrogenated in step (b) contain 1 to 5, preferably 1 to 3 halogen atoms, preferably selected from chlorine and fluorine atoms.

10. The process according to any one of claims 7 to 9, wherein the nitro compounds to be hydrogenated in step (b) are aliphatic or heteroaliphatic or heterocyclic nitro compounds containing a total of from 3 to 16 carbon atoms, 1 or 2 nitro groups, and optionally 1 to 3 further functional groups selected from hydroxyl groups and primary amino groups.

11. The process according to any one of claims 7 to 9, wherein the nitro compounds to be hydrogenated in step (b) are aromatic or heteroaromatic nitro compounds containing a total of from 6 to 14 carbon atoms, 1 or 2 nitro groups, and optionally 1 or 2 halogen atoms selected from chlorine and fluorine.

12. The process according to any one of claims 1 to 11, wherein the hydrogenation in step (b) is selected from

(1) hydrogenation of nitrobenzene to aniline;
(2) hydrogenation of nitrotoluene to toluidine;
(3) hydrogenation of dinitrotoluene to toluyldiamine;
(4) hydrogenation of dinitronaphthalene to diaminonaphthalene;
(5) hydrogenation of chloronitrobenzene to chloroaniline;
(6) hydrogenation of fluoronitrobenzene to fluoroaniline;
(7) hydrogenation of dichloronitrobenzene to dichloroaniline;
(8) hydrogenation of 2-nitro-2-methyl-1-propylamine to 2-methyl-1,2-diaminopropane;
(9) hydrogenation of 2-nitro-butanol to 2-amino-butanol;
(10) hydrogenation of 2-nitro-2-methylpropanol to 2-amino-2-methylpropanol;
(11) hydrogenation of 2-Nitro-2-methyl-1,3-propandiol to 2-amino-2-methyl-1,3-propandiol;
(12) hydrogenation of 2-nitro-2-ethyl-1,3-propandiol to 2-amino-2-ethyl-1,3-propandiol;
(13) hydrogenation of 2-nitro-2-hydroxymethyl-1,3-propandiol to 2-amino-2-hydroxymethyl-1,3-propandiol;
(14) hydrogenation of 1,1'-(ethane-1,2-diylbis(methylazanediyl))bis(4-nitro-4-methylpentan-2-ol) to 1,1'-(ethane-1,2-diylbis(methylazanediyl))bis(4-amino-4-methylpentan-2-ol);
(15) hydrogenation of 1,1'-(2-hydroxyethylazanediyl)bis(4-nitro-4-methylpentan-2-ol) to 1,1'-(2-hydroxyethylazanediyl)bis(4-amino-4-methylpentan-2-ol);
(16) hydrogenation of 5-nitro-1,3-dioxane derivatives to the corresponding 5-Amino-1,3-dioxane derivatives.

**13.** Amine compound, obtainable by the process according to any one of claims 1 to 12.

**14.** Amine compound according to claim 13, wherein the molar share of deuterium in the hydrogen bound in the primary amino group as a result of step (b) of the process according to any one of claims 1 to 12 is $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

**15.** Amine compound according to claim 13 or 14, wherein the amine compound is an aliphatic, heteroaliphatic, heterocyclic, aromatic or heteroaromatic amino compound containing a total of from 2 to 20 carbon atoms and from 1 to 3 primary amino groups.

**16.** Amine compound according to claim 15, wherein the amine compound contains from 1 to 5, preferably from 1 to 3 further functional groups selected from hydroxyl groups, carbonyl groups, nitrile groups and ether groups, and/or contains from 1 to 5, preferably from 1 to 3 halogen atoms, preferably selected from chlorine and fluorine atoms.

**17.** The use of the molar share of deuterium in downstream compounds based on hydrogen for tracing the origin, especially the energetic origin, of hydrogen bound in the downstream compounds based on hydrogen, wherein the downstream compounds are amine compounds.

**18.** The use according to claim 17, wherein the downstream compounds are amine compounds according to any one of claims 13 to 16.

**19.** A process for tracing the origin, especially the energetic origin, of hydrogen bound in downstream compounds based on hydrogen by determining the molar share of deuterium in said downstream compounds based on hydrogen, wherein the downstream compounds are amine compounds.

**20.** The process according to claim 19, wherein the downstream compounds are amine compounds according to any one of claims 13 to 16.

Europäisches Patentamt
European Patent Office
Office européen des brevets

# EUROPEAN SEARCH REPORT

Application Number
EP 24 20 7987

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/136097 A1 (SMAJLOVIC IVAN [RS]) 9 June 2011 (2011-06-09)<br>* the whole document * | 17-20 | INV.<br>C25B1/04<br>C25B15/08<br>C01B4/00<br>C07B59/00<br>C07C209/34<br>C07C209/36 |
| A | FELLOWS S K ET AL: "Availability of large quantities of low-deuterium hydrogen, and possible uses", INTERNATIONAL JOURNAL OF HYDROGEN ENERGY, ELSEVIER, AMSTERDAM, NL, vol. 6, no. 1, 1 January 1981 (1981-01-01), pages 67-71, XP025591252, ISSN: 0360-3199, DOI: 10.1016/0360-3199(81)90098-7 [retrieved on 1981-01-01]<br>* the whole document * | 17-20 | |
| X | US 2022/389150 A1 (BULAN ANDREAS [DE] ET AL) 8 December 2022 (2022-12-08)<br>* the whole document * | 1-20 | |
| X | GB 1 431 640 A (BAYER AG) 14 April 1976 (1976-04-14)<br>* the whole document * | 1-20 | TECHNICAL FIELDS SEARCHED (IPC)<br>C25B<br>C07B<br>C07C<br>C01C<br>C01B |
| A,P | WO 2023/213713 A1 (BASF SE [DE]) 9 November 2023 (2023-11-09)<br>* the whole document * | 17-20 | |

The present search report has been drawn up for all claims

1

| Place of search | Date of completion of the search | Examiner |
|---|---|---|
| Munich | 5 March 2025 | Ritter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 7987

This annex lists the patent family members relating to the patent documents cited in the above-mentioned European search report. The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| US 2011136097 A1 | 09-06-2011 | NONE | |
| US 2022389150 A1 | 08-12-2022 | CN 114599635 A | 07-06-2022 |
| | | EP 3819259 A1 | 12-05-2021 |
| | | EP 4054975 A1 | 14-09-2022 |
| | | JP 2023500262 A | 05-01-2023 |
| | | KR 20220098137 A | 11-07-2022 |
| | | US 2022389150 A1 | 08-12-2022 |
| | | WO 2021089737 A1 | 14-05-2021 |
| GB 1431640 A | 14-04-1976 | CH 598189 A5 | 28-04-1978 |
| | | DD 118415 A5 | 05-03-1976 |
| | | ES 432989 A1 | 16-02-1977 |
| | | FR 2254553 A1 | 11-07-1975 |
| | | GB 1431640 A | 14-04-1976 |
| | | JP S5093927 A | 26-07-1975 |
| | | NL 7416449 A | 20-06-1975 |
| WO 2023213713 A1 | 09-11-2023 | AU 2023266064 A1 | 14-11-2024 |
| | | CN 119156370 A | 17-12-2024 |
| | | EP 4519241 A1 | 12-03-2025 |
| | | KR 20250006960 A | 13-01-2025 |
| | | WO 2023213713 A1 | 09-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3953322 A1
- EP 0011090 B1
- EP 4021885 A1
- EP 0696573 B1
- CN 112108139
- CN 113019270
- EP 2382181 A
- EP 3055284 A, Covestro
- CN 101575295
- DE 2438542
- CN 103664642
- CN 104130129
- EP 0398542 A
- CN 103694124
- US 2012038336 A
- WO 2011115803 A1
- EP 0348223 A2
- US 8480800 B2
- EP 3674293 A1
- EP 24164893

### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31 395
- **H. SATO et al.** *International Journal of Hydrogen Energy*, 2021, vol. 46, 33 689-33 695
- **C.STOCK**. Hydrogenation and Dehydrogenation. *Ullmann's Encyclopedia of Industrial Chemistry*, 2023, https://doi.org/10.1002/14356007.a13_487.pub3
- B6champ Reduction. **NATTE, K.** ; **JAGADEESH, R.** Kirk-Othmer Encyclopedia of Chemical Technology, Chapter Amines by Reduction. John Wiley and Sons, 2021
- **WITTCOFF H. A.** ; **REUBEN, B. G.** ; **PLOTKIN, J. S.** Industrial Organic Chemicals. Wiley, 2004, 294
- Ullmann's Enzyklopädie der technischen Chemie. Verlag Chemie, 1973, vol. 7, 393
- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionization mass spectrometry (TIMS); A complementary quantitative technique to conventional mass resolved mass spectrometry. *Vacuum*, 2014, vol. 101, 416-422
- **W.A. BRANDT**. RAPID COMMUNICATIONS IN MASS SPECTROMETRY. *Rapid Commun. Mass Spectrom*, 1999, vol. 13, 1226-1230
- SNIF-NMR Part 1: Principles. **GÉRARD J. MARTIN** ; **SERGE AKOKA** ; **MARYVONNE L. MARTIN**. Modern Magnetic Resonance. Springer, 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008, 1669-1680